# EUROPEAN PATENT APPLICATION

(11) **EP 4 825 874 A1**
(43) Date of publication of application: **21.10.2026**
(21) Application number: 24918275.9
(22) Date of filing: 12.12.2024
(51) Int. Cl.: C07K 16/28, C12N 15/13, C12N 5/10, A61K 39/395, A61P 37/00, A61P 17/00

(54) **ANTI-HUMAN CD117 MONOCLONAL ANTIBODY AND USE THEREOF**

(30) Priority: 15.01.2024 CN 202410059200
(71) Applicant: Qyuns Therapeutics Co., Ltd., Taizhou, Jiangsu 225300 (CN)
(72) Inventor: QIU, Jiwan, Taizhou, Jiangsu 225300 (CN); KONG, Yong, Taizhou, Jiangsu 225300 (CN); CHEN, Wei, Taizhou, Jiangsu 225300 (CN); ZHOU, Yi, Taizhou, Jiangsu 225300 (CN); LIU, Yuzhi, Taizhou, Jiangsu 225300 (CN); LI, Wang, Taizhou, Jiangsu 225300 (CN); SUN, Rui, Taizhou, Jiangsu 225300 (CN); CHEN, Tao, Taizhou, Jiangsu 225300 (CN); QIAO, Huaiyao, Taizhou, Jiangsu 225300 (CN); WU, Yiliang, Taizhou, Jiangsu 225300 (CN)
(74) Representative: Lavoix
(86) International application number: PCT/CN2024/138805
(87) International publication number: WO 2025/152667

(57) **Abstract**

The present invention provides an anti-human CD117 monoclonal antibody and a use thereof. The anti-human CD117 monoclonal antibody of the present invention comprises three heavy chain complementarity-determining regions CDR-H1, CDR-H2 and CDR-H3 having amino acid sequences as shown in SEQ ID NOs. 1-3, respectively, and three light chain complementarity-determining regions CDR-L1, CDR-L2 and CDR-L3 having amino acid sequences as shown in SEQ ID NOs. 4-6, respectively. The antibody shows good activity at cytology level.

## Description

### TECHNICAL FIELD

The present application relates to the field of antibody drugs. Specifically, the present application relates to an anti-human CD117 monoclonal antibody and use thereof.

### BACKGROUND ART

Chronic urticaria (CU) is a common disease primarily driven by mast cells, with a significant negative impact on patients' quality of life. The signs and symptoms of CU are caused by the activation and degranulation of cutaneous mast cells (MCs), and the subsequent release of mediators that trigger sensory nerve activation, vasodilation, extravasation, and recruitment of circulating inflammatory cells (including eosinophils and basophils). Mast cells (MCs) are bone marrow-derived cells present in many organs and tissues of the human body. They contain a large number of pre-existing and newly formed secretory granules and possess unique pleiotropic properties. CD117 (also known as c-Kit) is a cell membrane protein encoded by the c-kit proto oncogene and belongs to the type III receptor tyrosine kinase family. It is primarily distributed on the surface of hematopoietic stem cells, mast cells, and most gastrointestinal stromal tumor cells, melanoma cells, and other cells. The CD117 protein contains 976 amino acids and is composed of an extracellular region, a transmembrane region, and a cytoplasmic region. The extracellular region includes five immunoglobulin-like (Ig) domains. The first three domains are important sites for CD117 to bind to its ligand, stem cell factor (SCF), while the last two domains are required for receptor dimerization. SCF is primarily secreted by endothelial cells, fibroblasts, and bone marrow stromal cells. Most SCF appears as a transmembrane protein, but the extracellular portion can be cleaved and released as a non-covalently linked dimer in solution. When CD117 binds to SCF, it homodimerizes and activates its intrinsic tyrosine kinase activity, subsequently triggering the initiation of multiple signaling pathways, including the phosphatidylinositol 3-kinase (PI3-K) pathway, the Janus kinase pathway, the signal transducer and activator of transcription (STAT) pathway, and the mitogen-activated protein kinase (MAPK) pathway. The physiological effects of c-Kit in controlling cell survival, proliferation, differentiation, and migration depend on the activation of specific or overlapping pathways, which confers complexity to the activity of the SCF/c-Kit system. When the c-kit gene is abnormally expressed in mast cells, the c-Kit protein can be spontaneously phosphorylated even in the absence of SCF binding, resulting in abnormal activation of the SCF/c-Kit signaling, regulating the maturation, migration, proliferation, degranulation, and release of inflammatory mediators of mast cells, mediating pathological inflammatory responses, and participating in the pathological processes of chronic inflammation and autoimmune diseases such as urticaria, nodular prurigo, and eosinophilic esophagitis.

Targeted inhibition of the SCF/c-Kit signaling pathway has promising applications in the prevention and treatment of spontaneous and induced urticaria, and holds enormous market potential. Related research indicates that Celldex Therapeutics' Barzolvolimab (CDX-0159), currently in Phase II clinical trials, is a humanized monoclonal antibody that binds with high specificity to the receptor tyrosine kinase CD117, which is essential for mast cell function and survival, and can potentially inhibit its activity. In a Phase II clinical trial for moderate to severe chronic spontaneous urticaria (CSU), it produced rapid, profound, and durable responses with a favorable safety, offering a new treatment option for patients with chronic urticaria.

### SUMMARY

The aim of this application is to provide a new anti-human CD117 monoclonal antibody, a pharmaceutical composition comprising the monoclonal antibody, and the use of the monoclonal antibody in the preparation of a medicament.

Specifically, the application involves the following contents:
1. An anti-human CD117 monoclonal antibody, comprising three heavy chain complementary determining regions CDR-H1, CDR-H2, and CDR-H3 and three light chain complementary determining regions CDR-L1, CDR-L2, and CDR-L3, wherein:
   the amino acid sequence of CDR-H1 (in the specification CDR-H1 represents heavy chain CDR1) is represented by SEQ ID NO: 1 (NKDVMG);
   the amino acid sequence of CDR-H2 (in the specification CDR-H2 represents heavy chain CDR2) is represented by SEQ ID NO: 2 (GIYTGSGSTYYASWAKG);
   The amino acid sequence of CDR-H3 (in the specification CDR-H3 represents heavy chain CDR3) is represented by SEQ ID NO: 3 (DLFYSNYYNL);
   the amino acid sequence of CDR-L1 (in the specification CDR-L1 represents light chain CDR1) is represented by SEQ ID NO: 4 (QASESISNYLS);
   the amino acid sequence of CDR-L2 (in the specification CDR-L2 represents light chain CDR2) is represented by SEQ ID NO: 5 (KASTLAS); and
   The amino acid sequence of CDR-L3 (in the specification CDR-L3 represents light chain CDR3) is represented by SEQ ID NO: 6 (QNTYVSSGSIT).
2. The monoclonal antibody according to item 1, wherein the monoclonal antibody comprises a heavy chain variable region and a light chain variable region, and wherein:
   the amino acid sequence of the heavy chain variable region is represented by SEQ ID NO: 7 (EVQLVESGGGLVQPGGSLRLSCAASGFSFSNKDVMGWVRQAPGKGLEWIAGIYTGSGSTY YASWAKGRFTISRDNSKNTLYLQMNSLRAEDTAVYYCARDLFYSNYYNLWGQGTLVTVSS) or has at least 90%, 91%, 92%, 93%, 94%, 95%, 96%, 97%, 98%, or 99% sequence identity with the amino acid sequence represented by SEQ ID NO: 7; and
   the amino acid sequence of the light chain variable region is represented by SEQ ID NO: 8 (DIQMTQSPSSVSASVGDRVTITCQASESISNYLSWYQQKPGKAPERLIYKASTLASGVPSRF SGSGSGTDFTLTISSLQPEDFATYYCQNTYVSSGSITFGGGTKVEIK) or has at least 90%, 91%, 92%, 93%, 94%, 95%, 96%, 97%, 98%, or 99% sequence identity with the amino acid sequence represented by SEQ ID NO: 8.
3. The monoclonal antibody according to item 1 or 2, wherein the monoclonal antibody comprises a heavy chain and a light chain, wherein:
   the amino acid sequence of the heavy chain is represented by SEQ ID NO: 10 (EVQLVESGGGLVQPGGSLRLSCAASGFSFSNKDVMGWVRQAPGKGLEWIAGIYTGSGSTY YASWAKGRFTISRDNSKNTLYLQMNSLRAEDTAVYYCARDLFYSNYYNLWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSVF LFPPKPKDTLYITREPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVF SCSVMHEALHNHYTQKSLSLSPGK) or has at least 90%, 91%, 92%, 93%, 94%, 95%, 96%, 97%, 98%, or 99% sequence identity with the amino acid sequence represented by SEQ ID NO: 10; and
   the amino acid sequence of the light chain is represented by SEQ ID NO: 11 (DIQMTQSPSSVSASVGDRVTITCQASESISNYLSWYQQKPGKAPERLIYKASTLASGVPSRF SGSGSGTDFTLTISSLQPEDFATYYCQNTYVSSGSITFGGGTKVEIKRTVAAPSVFIFPPSDEQL KSGTASVVCLLNNFYPREAKVQWKVDNALQSGNSQESVTEQDSKDSTYSLSSTLTLSKAD YEKHKVYACEVTHQGLSSPVTKSFNRGEC) or has at least 90%, 91%, 92%, 93%, 94%, 95%, 96%, 97%, 98%, or 99% sequence identity with the amino acid sequence represented by SEQ ID NO: 11.
4. An isolated nucleic acid encoding the monoclonal antibody according to any one of items 1 to 3.
5. A host cell comprising the nucleic acid according to item 4.
6. A method for producing a monoclonal antibody, comprising culturing the host cell according to item 5 to thereby produce the monoclonal antibody according to any one of items 1 to 3.
7. A pharmaceutical composition, comprising the monoclonal antibody according to any one of items 1 to 3 and a pharmaceutically acceptable carrier.
8. The pharmaceutical composition according to item 7, wherein the pharmaceutical composition is used to treat a disease related to signaling mediated by human CD117 protein.
9. The pharmaceutical composition according to item 8, wherein the disease related to signaling mediated by human CD117 protein comprises immune system diseases, digestive system diseases, and blood and lymphatic system diseases.
10. The pharmaceutical composition according to item 9, wherein the immune system disease comprises eosinophilic esophagitis, chronic urticaria and prurigo.
11. Use of the monoclonal antibody according to any one of items 1 to 3 in the preparation of a medicament for treating a disease related to signaling mediated by human CD117 protein.
12. The use according to item 11, wherein the a disease related to signaling mediated by human CD117 protein comprises immune system diseases, digestive system diseases, and blood and lymphatic system diseases, preferably, the immune system disease comprises eosinophilic esophagitis, chronic urticaria and prurigo.
13. A method for treating a disease related to signaling mediated by human CD117 protein, comprising a step of administering a therapeutically effective amount of the monoclonal antibody according to item 1 to a patient suffering from the disease.

### Effects of the invention

The present application provides a new anti-human CD117 monoclonal antibody, which has better affinity and biological activity for human CD117 protein than the CD117 monoclonal antibodies in the prior art, such as CDX-0159 (CDX-0159 is a monoclonal antibody drug targeting CD117 developed by Celldex Therapeutics, which can produce rapid, deep and lasting responses in a phase 1b clinical trial of moderate to severe chronic spontaneous urticaria and has good safety). The monoclonal antibody provided in this application exhibits superior biological activity at the cellular level compared to the CD117 monoclonal antibodies in the prior art, and is expected to demonstrate good clinical efficacy in the prevention and treatment of related diseases.

### BRIEF DESCRIPTION OF THE DRAWINGS

Fig. 1 is a schematic diagram of the nucleic acid electrophoresis results of the QX013N transient expression plasmid. Wherein M: Marker; Lane 1: 524VH-Hu35, HindIII/EcoRI; Lane 2: pQXHC, HindIII/EcoRI; Lane 3: PCR product 524VK-Hu17; Lane 4: pQX2.3, HindIII/BsiWI.
Fig. 2 is a flowchart of transient expression.
Fig. 3 is a schematic diagram showing the electrophoresis detection results of QX013N (HZD524-61).
Fig. 4 is a schematic diagram showing the results of the QX013N neutralizing activity analysis (cell proliferation method - M07e cells).
Fig. 5 is a schematic diagram showing the results of the QX013N neutralizing activity analysis (reporter gene method - NF-κB signaling).
Fig. 6 is a schematic diagram showing the results of the QX013N neutralizing activity analysis (FACS blocking method - LUVA cells).
Fig. 7 is a schematic diagram showing the binding activity of QX013N and CDX-0159 to porcine CD117.
Fig. 8 is a schematic diagram showing the signal value effect of QX013N and CDX-0159 neutralizing human SCF binding to human CD117.

The accompanying drawings are used to better understand the present application and do not constitute an improper limitation to the present application.

### DETAILED DESCRIPTION

The present application is described in further detail below in conjunction with specific embodiments. The examples provided in this application are intended to enable a more thorough understanding of the present application and to fully convey the scope of the present application to those skilled in the art. The various details of the examples are provided to aid understanding and should be considered as merely exemplary. Therefore, those skilled in the art should recognize that various changes and modifications can be made to the examples described herein without departing from the scope and spirit of the present application. Similarly, for clarity and conciseness, descriptions of well-known functions and structures have been omitted in the following description.

The technical terms mentioned in this specification have the same meanings as those commonly understood by those skilled in the art. In case of conflict, the definitions in this specification shall prevail.

Generally speaking, the terms used in this specification have the following meanings.

In this specification, "isolated" antibodies refer to antibodies that have been separated from their natural environmental components. In some embodiments, the antibody is purified to a purity greater than 95% or 99%, in which the purity is determined by methods such as electrophoresis (e.g., SDS-PAGE, isoelectric focusing (IEF), capillary electrophoresis) or chromatography (e.g., ion exchange or reverse phase HPLC). The method for evaluating antibody purity is known in the art, for example, the method may refer to Flatman et al, J. Chromatogr. B848: 79-87 (2007).

In this specification, "monoclonal antibody" refers to an antibody derived from a population of essentially homologous antibodies, that is, various antibodies constituting the population are identical and/or bind to the same epitope, except for possible variant antibodies (such as those containing naturally occurring mutations or produced during the production process of monoclonal antibody products), which typically exist in trace amounts. Unlike polyclonal antibody products that typically include different antibodies targeting different determinants (epitopes), each monoclonal antibody in monoclonal antibody products targets a single determinant on the antigen. Therefore, the modifier 'monoclonal' indicates that the antibody is derived from a substantially homologous antibody population and should not be interpreted as requiring the production of the antibody by any specific method. For example, the monoclonal antibody to be used in this application can be prepared using various techniques, including but not limited to hybridoma methods, recombinant DNA methods, phage display methods, and methods using genetically modified animals containing all or part of the human immunoglobulin gene locus. This application describes such methods and other exemplary methods for preparing monoclonal antibodies.

In this specification, "affinity" refers to the strength of the sum of non-covalent interactions between a single binding site of a molecule (e.g. antibody) and its binding partner (e.g. antigen). Unless otherwise specified, the term 'binding affinity' used in this specification refers to the inherent binding affinity reflecting the 1:1 interaction between members of a binding pair (e.g. antibody and antigen). The affinity of molecule X for its partner Y can usually be represented by the equilibrium dissociation constant (KD). Affinity can be measured by common methods known in this field.

In this specification, human CD-117 protein (sometimes also referred to as C-kit) refers to a membrane receptor derived from humans, and its extracellular amino acid sequence is represented by SEQ ID NO: 9, wherein the underlined portion represents the signal peptide.

SEQ ID NO: 9:
MRGARGAWDFLCVLLLLLRVQTGSSQPSVSPGEPSPPSIHPGKSDLIVRVGDEIRLLCT
DPGFVKWTFEILDETNENKQNEWITEKAEATNTGKYTCTNKHGLSNSIYVFVRDPAKLFLV
DRSLYGKEDNDTLVRCPLTDPEVTNYSLKGCQGKPLPKDLRFIPDPKAGIMIKSVKRAYHR
LCLHCSVDQEGKSVLSEKFILKVRPAFKAVPVVSVSKASYLLREGEEFTVTCTIKDVSSSVY
STWKRENSQTKLQEKYNSWHHGDFNYERQATLTISSARVNDSGVFMCYANNTFGSANVT
TTLEVVDKGFINIFPMINTTVFVNDGENVDLIVEYEAFPKPEHQQWIYMNRTFTDKWEDYP
KSENESNIRYVSELHLTRLKGTEGGTYTFLVSNSDVNAAIAFNVYVNTKPEILTYDRLVNG
MLQCVAAGFPEPTIDWYFCPGTEQRCSASVLPVDVQTLNSSGPPFGKLVVQSSIDSSAFKH
NGTVECKAYNDVGKTSAYFNFAFKGNNKEQIHPHTLFTP

In this specification, "anti-human CD117 monoclonal antibody" refers to a monoclonal antibody that can bind to human CD117 with sufficient affinity, making it suitable for use as a diagnostic and/or therapeutic agent targeting human CD117.

The anti-human CD117 monoclonal antibody in this application may not bind to proteins unrelated to the target. Herein, "proteins unrelated to the target" refer to proteins other than human CD117 as the target. Herein, "not bind" refers to that when the binding ability of the anti-human CD117 monoclonal antibody of the present application to the human CD117 as the target is taken as 100%, the binding ability of the anti-human CD117 monoclonal antibody of the present application to the unrelated protein is less than 10%, such as less than 5%, 1%, 0.1%, 0.01%, 0.001%, or 0.

The human CD117 monoclonal antibody of this application has an equilibrium dissociation constant (KD) of ≤1 µM, ≤100 nM, ≤50 nM, or ≤40 nM.

The experimental results show that the anti-human CD117 monoclonal antibody of this application can specifically bind to human CD117.

The anti-human CD117 monoclonal antibody of the present application can bind to CD-117 from, for example, marmosets, cynomolgus monkeys, rhesus monkeys, cats, dogs, and pigs, but may not bind to CD-117 from, for example, rats and mice.

The anti-human CD-117 monoclonal antibody of the present application is comparable to similar monoclonal antibody products reported, or superior to similar monoclonal antibody products on the market in many aspects of biological activity, including, for example, neutralizing the binding activity of recombinant human SCF to CD117.

Specifically, the present application relates to an anti-human CD117 monoclonal antibody, which comprises three heavy chain complementary determining regions CDR-H1 (heavy chain CDR1), CDR-H2 (heavy chain CDR2), and CDR-H3 (heavy chain CDR3), and three light chain complementary determining regions CDR-L1 (light chain CDR1), CDR-L2 (light chain CDR2), and CDR-L3 (light chain CDR3), wherein: the amino acid sequence of CDR-H1 is represented by SEQ ID NO: 1 (NKDVMG); the amino acid sequence of CDR-H2 is represented by SEQ ID NO: 2 (GIYTGSGSTYYASWAKG); the amino acid sequence of CDR-H3 is represented by SEQ ID NO: 3 (DLFYSNYYNL); the amino acid sequence of CDR-L1 is represented by SEQ ID NO: 4 (QASESISNYLS); the amino acid sequence of CDR-L2 is represented by SEQ ID NO: 5 (KASTLAS); and the amino acid sequence of CDR-L3 is represented by SEQ ID NO: 6 (QNTYVSSGSIT).

In a specific embodiment, the anti-human CD117 monoclonal antibody described herein comprises a heavy chain variable region HCVR and a light chain variable region LCVR, wherein:
the amino acid sequence of the heavy chain variable region is represented by SEQ ID NO: 7 (EVQLVESGGGLVQPGGSLRLSCAASGFSFSNKDVMGWVRQAPGKGLEWIAGIYTGSGSTY YASWAKGRFTISRDNSKNTLYLQMNSLRAEDTAVYYCARDLFYSNYYNLWGQGTLVTVSS) or has at least 80%, 81%, 82%, 83%, 84%, 85%, 86%, 87%, 88%, 89%, 90%, 91%, 92%, 93%, 94%, 95%, 96%, 97%, 98% or 99% sequence identity with the amino acid sequence represented by SEQ ID NO: 7; and
the amino acid sequence of the light chain variable region is represented by SEQ ID NO: 8 (DIQMTQSPSSVSASVGDRVTITCQASESISNYLSWYQQKPGKAPERLIYKASTLASGVPSRF SGSGSGTDFTLTISSLQPEDFATYYCQNTYVSSGSITFGGGTKVEIK) or has at least 80%, 81%, 82%, 83%, 84%, 85%, 86%, 87%, 88%, 89%, 90%, 91%, 92%, 93%, 94%, 95%, 96%, 97%, 98% or 99% sequence identity with the amino acid sequence represented by SEQ ID NO: 8.

In a specific embodiment, the anti-human CD117 monoclonal antibody described herein comprises a heavy chain and a light chain, wherein:
the amino acid sequence of the heavy chain is represented by SEQ ID NO: 10 (EVQLVESGGGLVQPGGSLRLSCAASGFSFSNKDVMGWVRQAPGKGLEWIAGIYTGSGSTY YASWAKGRFTISRDNSKNTLYLQMNSLRAEDTAVYYCARDLFYSNYYNLWGQGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSVF LFPPKPKDTLYITREPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVF SCSVMHEALHNHYTQKSLSLSPGK) or has at least 80%, 81%, 82%, 83%, 84%, 85%, 86%, 87%, 88%, 89%, 90%, 91%, 92%, 93%, 94%, 95%, 96%, 97%, 98% or 99% sequence identity with the amino acid sequence represented by SEQ ID NO: 10; and
the amino acid sequence of the light chain is represented by SEQ ID NO: 11 (DIQMTQSPSSVSASVGDRVTITCQASESISNYLSWYQQKPGKAPERLIYKASTLASGVPSRF SGSGSGTDFTLTISSLQPEDFATYYCQNTYVSSGSITFGGGTKVEIKRTVAAPSVFIFPPSDEQL KSGTASVVCLLNNFYPREAKVQWKVDNALQSGNSQESVTEQDSKDSTYSLSSTLTLSKAD YEKHKVYACEVTHQGLSSPVTKSFNRGEC) or has at least80%, 81%, 82%, 83%, 84%, 85%, 86%, 87%, 88%, 89%, 90%, 91%, 92%, 93%, 94%, 95%, 96%, 97%, 98% or 99% sequence identity with the amino acid sequence represented by SEQ ID NO: 11.
SEQ ID NO: 10 
EVQLVESGGGLVQPGGSLRLSCAASGFSFSNKDVMGWVRQAPGKGLEWIAGIYTGSG
STYYASWAKGRFTISRDNSKNTLYLQMNSLRAEDTAVYYCARDLFYSNYYNLWGQGTLVT
VSSASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKVEPKSCDKTHTCPPCPAPEAAGGP
SVFLFPPKPKDTLYITREPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGN
VFSCSVMHEALHNHYTQKSLSLSPGK
SEQ ID NO: 11

DIQMTQSPSSVSASVGDRVTITCQASESISNYLSWYQQKPGKAPERLIYKASTLASGVP
SRFSGSGSGTDFTLTISSLQPEDFATYYCQNTYVSSGSITFGGGTKVEIKRTVAAPSVFIFPPSD

EQLKSGTASVVCLLNNFYPREAKVQWKVDNALQSGNSQESVTEQDSKDSTYSLSSTLTLSK
ADYEKHKVYACEVTHQGLSSPVTKSFNRGEC

Among them, SEQ ID NOs: 10 and 11 are both humanized sequences. Through CDR grafting and back mutation of amino acids in the framework region that potentially affect the structure of the CDR regions, and after Fc modification, the activity is consistent with that of the maternal rabbit antibody, with a humanization degree of 97.0%. In this specification, "isolated" nucleic acid refers to a nucleic acid molecule that has been separated from its natural environmental components. Isolated nucleic acids include nucleic acid molecules typically present in cells comprising nucleic acid molecules, but the nucleic acid molecules exist outside the chromosome or at a chromosomal position different from their natural chromosomal position.

In this specification, 'isolated nucleic acid encoding anti-CD117 monoclonal antibody' refers to one or more nucleic acid molecules encoding the heavy and light chains of the antibody, including such nucleic acid molecules in a single vector or separate vectors, as well as such nucleic acid molecules present at one or more positions in the host cell.

In this specification, "vector" refers to a nucleic acid molecule that can amplify another nucleic acid connected to it. This term includes vectors that serve as self-replicating nucleic acid structures and vectors integrated into the genome of host cells into which they have been introduced. Some vectors can guide the expression of nucleic acids that are operationally linked to them. This type of vector is referred to as an "expression vector" in this application.

In this specification, "host cell", "host cell line", and "host cell culture" are interchangeable and refer to cells in which exogenous nucleic acid has been introduced, including the offspring of such cells. Host cells include "transformants" and "transformed cells", which include primary transformed cells and offspring derived from them (without considering the number of generations). Offspring may not be completely identical to parental cells in terms of nucleic acid content, and may contain mutations. The mutant offspring with the same function or biological activity selected or screened from the initial transformed cells are included in this specification.

In this specification, "pharmaceutical composition" refers to a product that presents in such a form that the biological activity of the active ingredient contained therein can exert the effect, and the composition does not contain any additional components that have unacceptable toxicity to the subject to whom the formulation is to be administered.

In this specification, "pharmaceutically acceptable carrier" refers to an ingredient in a pharmaceutical composition other than the active ingredient, which is non-toxic to the subject. Pharmaceutically acceptable carriers include, but are not limited to, buffering agents, excipients, stabilizers, or preservatives.

In this application, "monoclonal antibody" is generally a human antibody, which can be prepared using techniques known to those skilled in the art. For example, human antibodies are generally described in van Dijk, M. A. and van de Winkel, J. G., Curr. Opin. Pharmacol. 5: 368-374 (2001) and Lonberg, N., Curr. Opin. Immunol. 20:450-459(2008).

Antibodies can be prepared by administering immunogens to genetically modified animals that have been modified so that antigen attack stimulates the production of intact human antibodies or intact antibodies with human variable regions. These animals typically contain some or all of the human immunoglobulin loci, which replace endogenous immunoglobulin loci, or exist outside the chromosome or randomly integrated into the animal's body. In such transgenic mice, the endogenous immunoglobulin loci are generally inactivated. For a review of methods for obtaining human antibodies from transgenic animals, please refer to Lonberg, N. Nat. Biotech. 23:1117-1125 (2005). You can also refer to, for example, XENOMOUSE^{™} technology described in US Patent No. 6,075,181 and No. 6,150,584, HUMAB^{®} technology described in US Patent No. 5,770,429, K-MMOUSE^{™} technology described in US Patent No. 7,041,870, and VELOCIMOUSE^{®} technology described in US Patent Application Publication No. US 2007/0061900. The human variable region of the complete antibody generated by such animals can be further modified, for example, by combining with different human constant regions.

Human antibodies can also be prepared using hybridoma based methods. Human myeloma and mouse-human hybrid myeloma cells for producing human monoclonal antibodies have been described (see, for example, Kozbor, D., J. Immunol. 133:3001-3005 (1984); Brodeur, B. R. et al, Monoclonal Antibody Production Techniques and Applications, Marcel Dekker, Inc., New York(1987), pp. 51-63; Boerner, P., et al, J. Immunol. 147: 86-95 (1991). The human antibodies produced by human B cell hybridoma technology are also recorded in Li, J. et al, Proc. Natl. Acad. Sci. USA103: 3557-3562 (2006). Other methods include those recorded in, for example, US Patent No. 7,189,826 (which describes the generation of monoclonal human IgM antibodies from hybridoma cell lines) and Ni, Xiandai Mianyixue, 26(4); 265-268 (which describes human-human hybridoma). The human hybridoma technique (Trioma technique) is also recorded in Vollmers, H. P. and Brandlein, S., Histology and Histopathology 20: 927-937(2005); Vollmers, H. P. and Brandlein, S., Methods and Findings in Experimentaland Clinical Pharmacology 27:185-191(2005).

Human antibodies can also be generated by isolating variable domain sequences of Fv clones selected from phage display libraries derived from humans, and then combining such variable domain sequences with the desired human constant domain.

It is also possible to select human antibodies based on autoantibody libraries, that is, it is also possible to isolate human antibodies by screening the combinatorial library for one or more antibodies with the desired activity. For example, various methods for producing phage display libraries and screening such libraries for antibodies with desired binding characteristics are known in this field. This method is summarized in, for example, Hoogenboom, H. R. et al, Methods in Molecular Biology 178: 1-37 (2001) and further recorded in, for example, McCafferty, J. et al, Nature 348:552-554(1990); Clackson, T. et al, Nature 352:624-628(1991); Marks, J.D. et al, J. Mol. Biol. 222:581-597(1992); Marks, J. D. and Bradbury, A., Methods in Molecular Biology 248:161-175(2003); Sidhu, S. S., et al, J. Mol. Biol. 338:299-310(2004); Lee, C.V., et al, J. Mol. Biol. 340:1073-1093(2004); Fellouse, F. A., Proc. Natl. Acad. Sci. USA 101: 12467-12472(2004); and Lee, C. V. et al, J. Immunol. Methods 284:119-132(2004).

In some phage display methods, the complete sets of VH and VL genes are cloned by polymerase chain reaction (PCR) and randomly recombined in a phage library. Antigen binding phages are then screened in the phage library, as described in Winter, G. et al, Ann. Rev. Immunol. 12: 433-455 (1994). Bacteriophages typically display antibody fragments in the form of single chain Fv (scFv) fragments or Fab fragments. Libraries derived from immunological sources provide high-affinity antibodies against immunogens without the need to construct hybridomas. Alternatively, it is possible to clone the non-immune complete set (for example, from humans)to provide a single source of antibodies against a large number of nonself-antigens and self-antigens in the absence of any immunity, as described in Griffiths, A. D. et al, EMBO J, 12: 725-734(1993). Finally, unrearranged V gene segments can also be cloned from stem cells, and highly variable CDR3 regions can be encoded using PCR primers containing random sequences and rearranged in vitro to generate non-immune libraries, as described in Hoogenboom, H. R. and Winter, G., J. Mol. Biol. 227: 381-388 (1992). Patent publications describing human antibody phage libraries include, for example, US Patent No. 5,750,373 and US Patent Publication No. 2005/0079574, 2005/0119455, 2005/0266000, 2007/0117126, 2007/0160598, 2007/0237764, 2007/0292936, and 2009/0002360.

The antibody can also be a multispecific antibody, such as a bispecific antibody. Bispecific antibodies are monoclonal antibodies that have binding specificity for at least two different sites. The techniques used to generate multispecific antibodies include but are not limited to recombinant coexpression of two pairs of immunoglobulin heavy and light chains with different specificities (see Milstein, C. and Cuello, A. C., Nature 305:537-540 (1983); WO 93/08829; and Traunecker, A. et al, EMBO J. 10: 3655-3659 (1991)) and "joint-into-hole" engineering (see, for example, U.S. Patent No. 5,731,168). The multispecific antibodies also can be generated by engineered electrostatic manipulation effect for generating antibody Fc-heterodimer molecules (WO 2009/089004); crosslinking two or more antibodies or fragments thereof (see, for example, U.S. Patent No. 4,676,980 and Brennan, M. et al., Science 229: 81-83(1985)); using leucine zipper to generate bispecific antibodies (see, for example, Kostelny, S. A. et al, J. Immunol. 148: 1547-1553(1992)); using the "bi-antibody" technique for generating bispecific antibody fragments (see, for example, Holliger, P., et al, Proc. Natl. Acad. Sci. USA 90: 6444-6448(1993)); using single chain Fv (scFv) dimers (see e.g. Gruber, M. et al, J. Immunol. 152: 5368-5374(1994)); and preparing tri-specific antibodies (for example, as described in Tutt, A., et al., J. Immunol. 147: 60-69 (1991)).

The monoclonal antibodies in this application also include engineered antibodies with three or more functional antigen binding sites, including "octopus antibodies" (see, for example, US 2006/0025576).

The antibodies in this application may also include multispecific antibody described in WO2009/080251, WO2009/080252, WO2009/080253, WO2009/080254, WO2010/112193, WO2010/115589, WO2010/136172, WO2010/145792, WO2010/145793, WO2011/117330, WO2012/025525, WO2012/025530, WO2013/026835, WO2013/026831, WO2013/164325 or WO2013/174873.

The monoclonal antibodies in this application can also be antibody variants, for example, it may be desirable to improve the binding affinity and/or other biological characteristics of the antibody. Amino acid sequence variants of the antibody can be prepared by introducing appropriate modifications into the nucleotide sequence encoding the antibody, or by peptide synthesis. Such modification includes, for example, deletion, insertion, and/or substitution of residues within the amino acid sequence of the antibody. Any combination of deletion, insertion, and substitution can be performed to obtain the final construct, as long as the final construct has the desired features, such as antigen binding. Therefore, in some embodiments, antibody variants with one or more amino acid replacements are provided, and sites of interest for replacement mutations include HVR and FR, for example, amino acid replacements can be introduced into the antibody of interest and products with desired activity (such as retained/improved antigen binding, reduced immunogenicity, or improved ADCC or CDC) can be selected.

### EXAMPLES

The content of this application will be described below with reference to specific examples, but the scope of this application is not limited thereto.

Unless otherwise specified, the reagents and instruments used in the following examples are conventional reagents and instruments in the art and can be obtained commercially. The experimental methods used are all conventional methods unless otherwise specified. Those skilled in the art can undoubtedly implement the described protocols and obtain the corresponding results based on the content of the examples.

### Example 1: Preparation of anti-human CD117 monoclonal antibody QX013N

The human CD117 antigen expressed and prepared by Qyuns Therapeutics Co., Ltd was used as an immunogen to immunize New Zealand rabbits. Monoclonal antibodies with antigen binding specificity were obtained using B cell cloning technology, and monoclonal antibodies that bound to human CD117 and inhibit human CD117 were selected. Binding ELISA and blocking ELISA assays are used for analysis and screening, and target clones are selected. The above immunization and screening processes were entrusted to commercial companies for completion.

Five clones were selected for recombinant expression and sequenced. After testing, it was found that clone 524 had the best cell neutralization activity. Therefore, clone 524 was humanized. The homology alignment of human IgG germline sequence was performed using NCBI IgBlast. IGHV3-66 * 01 was selected as the template for heavy chain CDR transplantation, and the CDR regions of the heavy chain of the clone 524 (i.e., CDR-H1 (SEQ ID NO: 1), CDR-H2 (SEQ ID NO: 2), and CDR-H3 (SEQ ID NO: 3)) were transplanted into the backbone region of IGHV3-66 * 01. IGKV1-12 * 01 was selected as the template for light chain CDR transplantation, and the CDR regions of the light chain of the clone 524 (i.e., CDR-L1 (SEQ ID NO: 4), CDR-L2 (SEQ ID NO: 5), and CDR-L3 (SEQ ID NO: 6)) were transplanted into the backbone region of IGKV1-12 * 01. Back mutations were introduced at specific sites in the backbone region and site directed mutagenesis was performed in CDR regions to obtain the variable region of monoclonal antibody QX013N in this application. Finally, the sequence of the humanized heavy chain variable region is represented by SEQ ID NO: 7; and the amino acid sequence of the humanized light chain variable region is represented by SEQ ID NO: 8.

The gene for the above heavy chain (SEQ ID NO: 10) was synthesized by GenScript and obtained by double digestion with HindIII and EcoRI. The gene for the light chain variable region (SEQ ID NO: 8) was obtained by PCR amplification. The heavy chain expression plasmid pQXHC was double-digested with HindIII and EcoRI. The light chain expression plasmid pQX2.3 was double-digested with HindIII and BsiWI. The heavy chain gene was inserted into the heavy chain expression plasmid using T4 ligase to construct the heavy chain expression plasmid pQXHC-524VH-Hu35. The PCR-amplified light chain gene was inserted into the light chain expression plasmid using infusion recombinase to construct the light chain expression plasmid pQX2.3-524VK-Hu17.

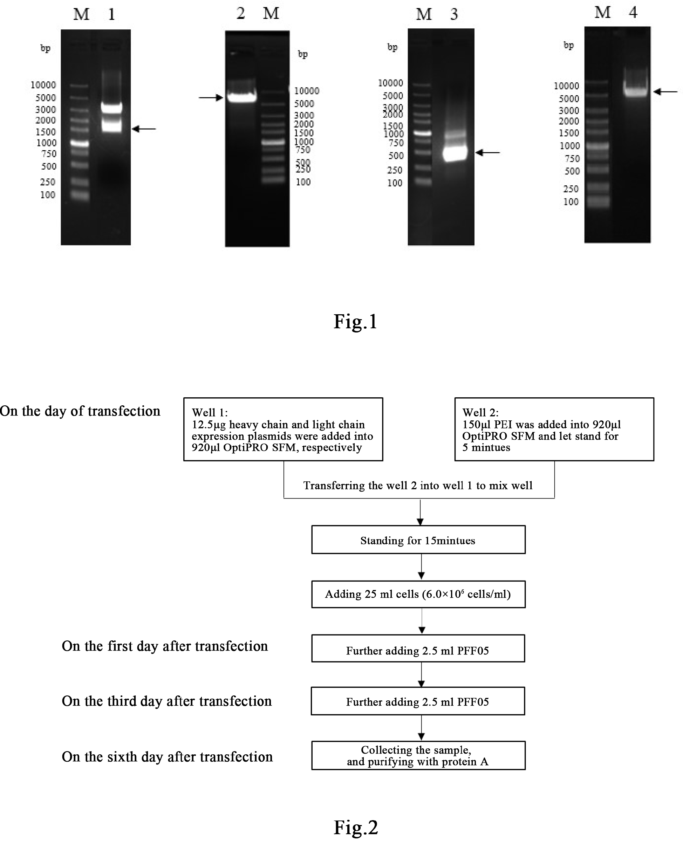

The results of nucleic acid electrophoresis of double-digested heavy chain gene fragments, PCR-amplified light chain variable region gene fragments, and double-digested plasmids are shown in Fig. 1. From Fig. 1, the double digestion results of the antibody heavy chain, the PCR amplification results of the light chain variable region, and the results of the double-digested heavy chain and light chain expression plasmids can be seen, in which the plasmid size of the heavy chain and light chain is about 5000 bp, with the full-length heavy chain being about 1410 bp, and the light chain variable region being about 447 bp.

ExpiCHO-S cells were co-transfected with the correctly sequenced heavy chain expression plasmid pQXHC-524VH-Hu35 (the amino acid sequence of the expressed heavy chain is represented by SEQ ID NO: 10) and light chain expression plasmid pQX2.3-524VK-Hu17 (the amino acid sequence of the expressed light chain is represented by SEQ ID NO: 11). On the day before transfection, ExpiCHO-S cells were diluted to 3 × 10⁶ cells/mL for passage. On the day of transfection, the cells were diluted to 6 × 10⁶ cells/mL, and 25 mL of cells were placed into a 125 mL of shake flask, ready for transfection. The transfection and expression processes are shown in Fig. 2.

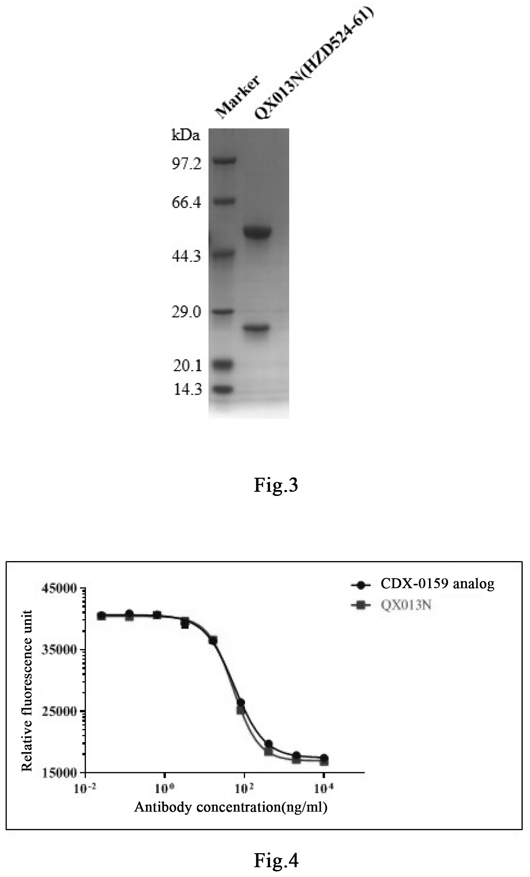

On the 6th day after transfection, the culture supernatant was harvested and purified in one step using ProteinA. The purified antibody was detected by SDS-PAGE electrophoresis and named QX013N(HZD524-61). The detection results of this antibody by protein electrophoresis are shown in Fig. 3. Protein electrophoresis was performed using denaturing reducing gel, and the results in Fig. 3 show two lanes with sizes of approximately 50 kDa and 25 kDa, respectively, which are consistent with the theoretical molecular weights of the heavy chain (49.2 kDa) and light chain (23.4 kDa).

### Example 2: Determination of Equilibrium Dissociation Constant (K_{D})

The affinity of QX013N(HZD524-61) with human CD117 was detected using Biacore T200, and all processes were performed at 25°C. An appropriate amount of antibody was fixed by capture method using a commercial Sensor chip CM5 chip, so that the Rmax was about 50 RU, and the capture flow rate was 10 µL/min. The antigen was gradiently diluted, and the flow rate in the instrument was switched to 30µL/min. The antigen flowed through the reference channel and the channel of the fixed antibody in order of concentration from low to high, with flowing through the buffer as a negative control. After each binding and dissociation was completed, the chip was regenerated with glycine of pH 1.5. The 1:1 binding model in the Kinetics option of the built-in analysis software of the instrument was selected for fitting, and the binding rate constant *kₐ,* dissociation rate constant *k_{d},* and dissociation equilibrium constant *K_{D}* of the antibody were calculated.

In addition, the affinity of QX013N(HZD524-61) was compared with that of CDX-0159, a monoclonal antibody against human CD117 currently in clinical phase II. The detection method for known antibody was the same as that for QX013N, and the results are shown in Table 1. CDX-0159 was obtained by constructing an expression plasmid based on the sequence provided by patent NZ630363B and transiently transfecting it into ExpiCHO-S cells.

**Table 1 Affinity of the anti-human CD117 monoclonal antibody binding to human CD117**

| Sample name | Kₐ(10⁵M⁻¹S⁻¹) | K _{d}(10⁻⁴S⁻¹) | K_{D}(10⁻⁹M) |
|---|---|---|---|
| CDX-0159 | 2.51 | 5.94 | 2.49 |
| QX013N (HZD524-61) | 2.02 | 2.51 | 1.40 |

The data in the table is: the calculated average value after three times of detection of each sample

### Example 3: neutralizing activity of QX013N and CDX-0159 against recombinant human SCF induced M07E cell proliferation

M07e cells were used to determine the neutralizing activity of QX013N and CDX-0159 against recombinant human SCF-induced cell proliferation. M07E cells were seeded into 96-well plates at 4×10⁴ cells per well. Antibodies were diluted to a concentration range of 0 to 10 µg/mL, added to the cells, and incubated for 1 hour. Subsequently, 50 ng/mL of recombinant human SCF was added, mixed, and cultured for 24 hours at 37°C with 5% CO₂. 100 µL of the cell suspension was collected and transferred to a black 96-well plate. Then, 100 µL of Cell Counting-Lite 2.0 Luminescent Cell Viability Assay reagent (Vazyme, DD1101-02) was added and mixed with the cell suspension. Cell proliferation was measured using a multifunctional microplate reader, and dose-response curves were plotted (as shown in Fig. 4) to analyze the neutralizing activity of the antibodies.

The results shown in Fig. 4 indicate that QX013N possesses neutralizing activity against recombinant human SCF-induced M07e cell proliferation, with an IC₅₀ value of 50.55 ng/mL, and the IC₅₀ value of the control antibody CDX-0159 is 55.11 ng/mL. The neutralizing activity of QX013N against human SCF-induced M07e cell proliferation is superior to that of the control antibody CDX-0159.

### Example 4: neutralizing activity of QX013N and CDX-0159 against recombinant human SCF induced NF-κB signaling in HEK293 reporter gene cells overexpressing human CD117

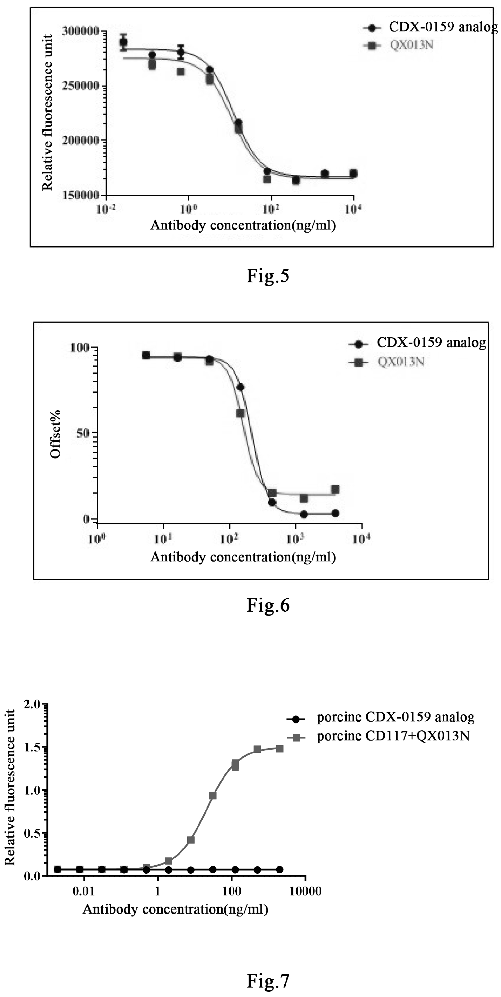

HEK293 reporter gene cells overexpressing human CD117 were used to determine the neutralizing activity of QX013N and CDX-0159 against recombinant human SCF-induced intracellular NF-κB signaling mediated by CD117. Human CD117-overexpressing HEK293 cells were seeded into 96-well plates at 4×10⁵ cells per well and cultured overnight at 37°C with 5% CO₂. Antibodies were diluted to a concentration range of 0 to 40 µg/mL, added to the cells, and incubated for 1 hour. Subsequently, 400 ng/mL of recombinant human SCF was added, and the cells were cultured for 24 hours at 37°C with 5% CO₂. The One-Lite Luciferase Assay System reagent (Vazyme, DD1203-02) was added to detect cells. Four-parameter logistic curves were fitted using SoftMax to calculate the IC₅₀ values and analyze the neutralizing activity of the antibodies. The results are shown in Fig. 5.

The results shown in Fig. 5 indicate that QX013N can neutralize recombinant human SCF-induced NF-κB signaling in HEK293 reporter gene cells overexpressing human CD117, with an IC₅₀ value of 9.70 ng/mL, and the IC₅₀ value of the control antibody CDX-0159 is 13.63 ng/mL. The neutralizing activity of QX013N against human SCF-induced reporter gene is superior to that of the control antibody CDX-0159.

### Example 5: neutralizing activity of QX013N and CDX-0159 against the binding of recombinant human SCF to CD117 on the cell membrane surface

LUVA cells were used to determine the neutralizing activity of QX013N and CDX-0159 against the binding of recombinant human SCF to CD117 on the cell surface membrane. LUVA cells were seeded into U-bottom 96-well plates at 5×10⁵ cells per well. Antibodies were diluted to a concentration range of 0 to 8 µg/mL, added to the cells, and incubated for 1 hour at 4°C. Subsequently, 20 µL of Bio SCF (10 µg/mL) was added to each well and incubated for 1 hour. Cells were washed twice with PBS, followed by the addition of Anti-Biotin eBioscience^{™} Alexa Fluor 488 detection reagent. After washing with PBS, cells were resuspended and analyzed using a flow cytometer. Four-parameter logistic curves were fitted using GraphPad to calculate the IC₅₀ values and analyze the neutralizing activity of QX013N against the binding of recombinant human SCF to CD117 on the membrane surface of LUVA cells. The results are shown in Fig. 6.

The results shown in Fig. 6 indicate that QX013N can neutralize the binding of recombinant human SCF to CD117 on the membrane surface of LUVA cells, with an IC₅₀ of 175.9 ng/mL, and the IC₅₀ value of the control antibody CDX-0159 is 223.4 ng/mL. The neutralizing activity of QX013N against the binding of recombinant human SCF to CD117 on the membrane surface of LUVA cell is superior to that of the control antibody CDX-0159.

### Example 6: Detection of binding activity of QX013N and CDX-0159 to CD117 from other species

Binding ELISA was used to detect the binding activity of QX013N and CDX-0159 to porcine CD117. Porcine CD117 was diluted to 1 µg/mL, added to ELISA plates, and coated overnight at 2-8°C. After blocking, a series of antibody dilutions ranging from 0 to 2 µg/mL and Goat-anti-human IgG HRP were added and incubated sequentially. Finally, substrate was added for color development, and the reaction was stopped by adding stop solution. After termination, the absorbance at 450 nm (with 650 nm as reference) was read using a microplate reader. Dose-response curves were plotted to analyze the binding specificity of the antibodies. The dose-response curve is shown in Fig. 7.

The results shown in Fig. 7 indicate that QX013N can bind to porcine CD117, whereas CDX-0159 does not bind. This result suggests that the epitopes recognized by QX013N and CDX-0159 are different.

Using the same method, we also tested the binding activity of QX013N to CD117 from other species. The experimental results show that QX013N binds to CD117 from marmoset, cynomolgus monkey, rhesus monkey, cat, and dog, but does not bind to CD117 from rat or mouse.

### Example 7: Difference in signal values for the neutralization of human SCF binding to human CD117 by QX013N and CDX-0159

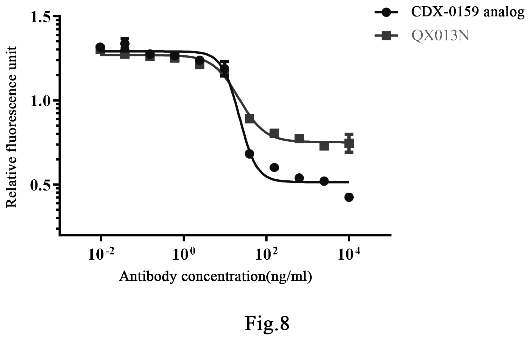

ELISA was used to detect the difference in signal values for the neutralization of human SCF binding to human CD117 by QX013N and CDX-0159. Human SCF was diluted to 1 µg/mL, added to ELISA plates, and coated overnight. A series of antibody dilutions ranging from 0 to 20 µg/mL were mixed with Bio Human CD117 diluted to 200 ng/mL and incubated. After incubation, the antigen-antibody mixtures were added to the blocked ELISA plates and incubated. Finally, substrate was added for color development, and the reaction was stopped by adding stop solution. After termination, the absorbance at 450 nm (with 650 nm as reference) was read using a microplate reader. Signal value response curves were plotted to analyze the differences in signal values for the neutralization of human SCF binding to human CD117 by the antibodies. The signal value response curve is shown in Fig. 8.

The results shown in Fig. 8 indicate that when the antibody concentration is 0, the binding signal value of human CD117 to human SCF is 100%; when the antigen-antibody binding reaches saturation, the signal value for the neutralization of human SCF binding to human CD117 by QX013N is 55.6%; the signal value for neutralization by CDX-0159 is 30.5%. This result suggests that the epitopes recognized by QX013N and CDX-0159 are different.

The foregoing descriptions are merely preferred examples of this application and are not intended to limit this application in any form. Any person skilled in the art may utilize the technical content disclosed above to alter or modify it into equivalent examples with equivalent changes. However, any simple modification, equivalent change, or alteration made to the above examples based on the technical essence of this application, without departing from the content of the technical solution of this application, shall still fall within the protection scope of the technical solution of this application.

## Claims

1. An anti-human CD117 monoclonal antibody, comprising three heavy chain complementarity determining regions CDR-H1, CDR-H2 and CDR-H3, and three light chain complementarity determining regions CDR-L1, CDR-L2 and CDR-L3, wherein:
the amino acid sequence of the CDR-H1 is represented by SEQ ID NO: 1;
the amino acid sequence of the CDR-H2 is represented by SEQ ID NO: 2;
the amino acid sequence of the CDR-H3 is represented by SEQ ID NO: 3;
the amino acid sequence of the CDR-L1 is represented by SEQ ID NO: 4;
the amino acid sequence of the CDR-L2 is represented by SEQ ID NO: 5; and
the amino acid sequence of the CDR-L3 is represented by SEQ ID NO: 6.

2. The monoclonal antibody according to claim 1, wherein the monoclonal antibody comprises a heavy chain variable region and a light chain variable region, and wherein:
the amino acid sequence of the heavy chain variable region is represented by SEQ ID NO: 7 or has at least 90%, 91%, 92%, 93%, 94%, 95%, 96%, 97%, 98%, or 99% sequence identity with the amino acid sequence represented by SEQ ID NO: 7; and
the amino acid sequence of the light chain variable region is represented by SEQ ID NO: 8 or has at least 90%, 91%, 92%, 93%, 94%, 95%, 96%, 97%, 98%, or 99% sequence identity with the amino acid sequence represented by SEQ ID NO: 8.

3. The monoclonal antibody according to claim 1 or 2, wherein the monoclonal antibody comprises a heavy chain and a light chain, wherein:
the amino acid sequence of the heavy chain is represented by SEQ ID NO: 10 or has at least 90%, 91%, 92%, 93%, 94%, 95%, 96%, 97%, 98%, or 99% sequence identity with the amino acid sequence represented by SEQ ID NO: 10; and
the amino acid sequence of the light chain is represented by SEQ ID NO: 11 or has at least 90%, 91%, 92%, 93%, 94%, 95%, 96%, 97%, 98%, or 99% sequence identity with the amino acid sequence represented by SEQ ID NO: 11.

4. An isolated nucleic acid encoding the monoclonal antibody according to any one of claims 1 to 3.

5. A host cell comprising the nucleic acid according to claim 4.

6. A method for producing a monoclonal antibody, comprising culturing the host cell according to claim 5 to produce the monoclonal antibody according to any one of claims 1 to 3.

7. A pharmaceutical composition, comprising the monoclonal antibody according to any one of claims 1 to 3 and a pharmaceutically acceptable carrier.

8. Use of the monoclonal antibody according to any one of claims 1 to 3 in the preparation of a medicament for treating a disease related to signaling mediated by human CD117 protein.

9. A method for treating a disease related to signaling mediated by human CD117 protein, comprising a step of administering a therapeutically effective amount of the monoclonal antibody according to claim 1 to a patient suffering from the disease.
